# EUROPEAN PATENT APPLICATION

(11) **EP 4 700 384 A1**
(43) Date of publication of application: **25.02.2026**
(21) Application number: 24818573.8
(22) Date of filing: 31.05.2024
(51) Int. Cl.: G01N 27/22, A61B 5/00

(54) **BODY DIFFUSIBLE SUBSTANCE MEASUREMENT DEVICE**

(30) Priority: 07.06.2023 CN 202310671538; 07.06.2023 CN 202321447748 U
(71) Applicant: Beijing Tashan Technology Co., Ltd., Beijing 102308 (CN)
(72) Inventor: SUN, Tengchen, Beijing 102308 (CN); WANG, Kai, Beijing 102308 (CN)
(74) Representative: Yang, Shu
(86) International application number: PCT/CN2024/096664
(87) International publication number: WO 2024/251041

(57) **Abstract**

The present invention relates to a device for measuring a substance diffused from a body, comprising a measuring chamber, a sensor, and an evaluation device. The measuring chamber has at least one top opening for discharge and a bottom opening for direct or indirect contact with the body, wherein the area of the top opening is configured to be smaller than the bottom opening. The measuring chamber is also provided with at least one third openings, wherein the third openings are used to form the bottom open structure of the measuring chamber during measurement; the sensor is located on the peripheral wall of the measuring chamber and is used to measure the concentration of the substance diffused by the body; the evaluation device is coupled to the sensor.

## Description

### Technical Field

present invention relates to a method for measuring a substance diffused by the body, and in particular to the detection of water evaporation from the skin.

For skin moisture testing, the most commonly used instrument in the market for beauty or medical purposes is the German CK skin water loss (TEWL) tester. The relevant patented technology is shown in CN202080026313.8. It uses a cylindrical measuring chamber with two openings of the same size on the upper and lower sides . Sensors that measure the concentration, temperature and humidity of diffusing substances (such as water vapor) are arranged on the inner wall of the measuring chamber to obtain the temperature distribution and water vapor flow distribution at various locations in the measuring chamber. The evaluation device uses the values measured by the sensors to measure water vapor based on the theory of material or energy diffusion.

The diffusion theory employed by the CK tester, known as Fick 's diffusion law, is based on a stable environment and requires steady airflow. (Diffusion includes both diffusion in stagnant air and diffusion under convective airflow.) However, the cylindrical measurement chamber of the CK Skin Water Loss (TEWL) tester has the same bottom and top openings. The bottom opening contacts the skin, creating a closed bottom and open top structure. Because human skin temperature is approximately 36 °C, while the top opening has an ambient temperature of approximately 20 °C, this temperature difference creates upward and downward convection currents, with heat flowing out and back in through the top opening, resulting in unstable airflow in the measurement chamber. To mitigate the impact of this conflict, the CK tester requires at least three sensors to be placed in the measurement chamber to measure densely packed objects. These sensors are positioned at varying distances from the object being tested. In practice, the CK employs five layers of six sensors per layer, for a total of 30 sensors, to detect temperature and humidity gradients at varying heights within the chamber. The system then indirectly calculates water evaporation using averaging and diffusion theory. However, the test results still exhibit significant discrepancies with the actual evaporation rate due to the neglect of convection. Furthermore, the complex structure of the multi-layer sensor system also increases costs.

### Summary of the Invention

The present invention aims to improve the deficiencies of the prior art and provides a device for measuring body diffusion substances.

The measuring device of the present invention includes a measuring chamber, a sensor, and an evaluation device. The measuring chamber has at least one top opening for discharge and a bottom opening for direct or indirect contact with the body, wherein the area of the top opening is configured to be smaller than the bottom opening. The measuring chamber is also provided with at least one third opening, and the third opening is used to form a bottom-open structure of the measuring chamber during measurement; the sensor is located on the peripheral wall of the measuring chamber and is used to measure the concentration of substances diffused from the body; the evaluation device is coupled to the sensor.

The present invention configures the area of the top opening of the measuring chamber to be smaller than the bottom opening, and provides a third opening at the bottom of the measuring chamber to form an open-bottom structure, so that the airflow in the measuring chamber is stable and upward, avoiding up and down convection, fundamentally resolving the contradiction and conflict with the theoretical basis of the diffusion law, and improving measurement accuracy.

In the present invention, the peripheral wall may include the outer wall and / or the interior of the measurement chamber, wherein the preselected sensor is arranged on the inner wall to increase the sensitivity.

The measuring device of the present invention can be used to detect the concentration of various diffusible substances, and is particularly suitable for detecting skin moisture evaporation. Taking moisture evaporation detection as an example, as an improved solution, since the evaporated water vapor flow is concentrated out of the small opening at the top, sensors can be arranged around the top opening of the measuring chamber, facilitating measurement of the cross-section at the top opening. Furthermore, since a stable airflow is formed within the measuring chamber, the number of sensors can be configured to form a single-layer sensor structure arranged around the top opening, thereby simplifying the structure and reducing costs. Alternatively, the number of sensors can be configured to be at least two, each at a different distance from the body during measurement, thereby forming a multi-layer sensor structure with varying heights around the top opening, further improving measurement accuracy.

As a further improvement of the improved solution, the present invention can be configured to achieve the measurement of diffused substances by means of capacitance. Specifically, the sensor is configured to include at least two electrodes, and the evaluation device is provided with a capacitance-to-digital conversion circuit (CDC) and a processing module. The capacitance-to-digital conversion circuit couples each electrode to obtain mutual capacitance. For the processing module, since water vapor passes through the mutual capacitance electric field, the dielectric in the electric field changes, resulting in a change in mutual capacitance. Therefore, the processing module can reflect the content of water vapor passing through the mutual capacitance electric field based on the change in mutual capacitance, and then output the concentration information of the substance diffused by the body. The present invention configures the sensor as at least two electrodes. On the one hand, through capacitive cooperation CDC , e.g. DAI 7142, AD I 7147,

A method that uses Δ-Σ modulation to charge and discharge the measured capacitor multiple times and compare it with the reference capacitor ( See: US Patent Number: 5,134,401) directly converts the measured capacitance value into a digital value, which can improve the measurement sensitivity of capacitance to the 1ff level. Furthermore, due to the narrowing of the top opening, the spacing between the electrodes arranged around the top opening is reduced, and the electrodes are closer to each other. According to the principle of capacitance, the sensitivity and detection accuracy of mutual capacitance can be improved. On this basis, further, the sensor can be configured to include at least three electrodes, and the capacitance-to-digital conversion circuit couples each electrode through a switch array, and is used to use each electrode in the sensor as an excitation in turn to obtain the mutual capacitance between it and other electrodes in the layer. For example: for a solution in which there are three electrodes A, B, and C in the sensor , electrode A is used as the excitation in the first measurement to obtain the mutual capacitance between electrodes AB and electrodes AC , and electrode B is used as the excitation in the second measurement to obtain the mutual capacitance between electrodes BC ; for another example, for a solution in which there are four electrodes A , B , C , and D in the sensor, electrode A is used as the excitation in the first measurement to obtain the mutual capacitance between electrodes AB, electrodes AC , and electrodes AD , electrode B is used as the excitation in the second measurement to obtain the mutual capacitance between electrodes BC and electrodes BD , and electrode C is used as the excitation in the third measurement to obtain the mutual capacitance between electrodes CD , and so on. Through this dynamic and synchronous detection of multiple pairs of mutual capacitances, the detection accuracy and sensitivity can be further improved.

Furthermore, since the smaller the ratio of the area of the top opening to the area of the bottom opening is, the greater the water vapor flow rate at the top opening will be, the detection sensitivity requirements of the sensor will be increased, and the detection accuracy results will be affected. Through a large number of experiments, we have configured the ratio of the area of the top opening to the bottom opening to be less than 0.6, thereby taking into account various aspects such as sensitivity, accuracy, and flow rate.

Furthermore, the top opening is configured as a flat hole, and the electrodes are distributed on the flat edges on both sides of the flat hole. In this solution, the top opening area remains unchanged while the spacing between the electrodes can be further reduced, thereby significantly optimizing the detection accuracy and sensitivity. And / or, the top opening can also be configured to include at least two, and the processing module is used to calculate the corresponding concentration information based on the collected data of the sensor at each top opening and perform a weighted average. By providing multiple openings, the total top opening area remains unchanged while the spacing between the electrodes at each opening is reduced, and the weighted average is then used to further improve the accuracy.

The temperature difference between the ambient temperature at the top opening and the body temperature (the human skin temperature in the experimental environment is about 36 degrees Celsius, the ambient temperature at the top opening is approximately 20 degrees Celsius, and the temperature difference between the two is more than ten degrees Celsius. This affects the flow rate (temperature and humidity also affect mutual capacitance in capacitive sensing solutions), and body temperatures vary slightly between different bodies (e.g., humans). Therefore, as another improved solution, a top temperature sensor for collecting the ambient temperature at the top opening and a bottom temperature sensor for collecting the body temperature during measurement can be provided in the measurement chamber. The evaluation device is coupled to the top and bottom temperature sensors, respectively, and is used to correct the concentration of the substance diffused from the body based on the temperature difference collected by the two, thereby eliminating the influence of temperature difference fluctuations during measurement. Furthermore, because the temperature and humidity of different environments also vary, the measurement baseline of the environment varies, introducing measurement errors. To this end, the body-diffused substance measurement device is also equipped with an ambient temperature sensor and / or ambient humidity sensor located outside the measurement chamber. The evaluation device is coupled to the ambient temperature sensor and / or ambient humidity sensor, and is used to correct the concentration of the substance diffused from the body based on the data collected by the ambient temperature sensor and/or ambient humidity sensor, thereby eliminating the interference caused by changes in ambient temperature and humidity.

As another improved solution, in the present invention, the top opening is located on the top surface of the measuring chamber, and the bottom opening is located on the bottom surface of the measuring chamber. The projection of the top opening is within the projection area of the bottom opening. By vertically aligning the top and bottom openings, a more stable and uncluttered airflow is achieved. Furthermore, the measuring chamber has a tapered structure that gradually tapers from the bottom to the top.

In the present invention, the third opening can be used to form a bottom-open structure in the measurement chamber during measurement in a variety of ways. For example, by connecting the bottom of the third opening to the bottom opening and setting the third opening large enough so that when the bottom opening is pressed against a body, such as a human body, the elastic skin of the body is insufficient to completely block the third opening, thereby achieving the purpose of bottom opening. In this solution, the elasticity of the skin of each body is different, and the blocking of the third opening will also be different, so that the difference in wind speed entering through the third opening will also introduce an impact on the measurement. We hope that the area of the bottom opening is as consistent as possible for each measurement. Therefore, it is preferable to set the lower edge of the third opening to a distance from the bottom opening, thereby eliminating the possibility of the body's skin falling into the third opening and maintaining the stability of the bottom opening area for each measurement. In the present invention, the lower edge of the third opening is at least 1 mm away from the bottom opening.

In the present invention, the height of the measuring chamber is configured to be greater than 1 cm so that the water vapor emitted from the body ca Steady rise.
FIG1 shows a schematic structural diagram of a device for measuring body diffusion substances;
FIG2 shows a cross-sectional view of the structure of a device for measuring body diffusion substances;
Figure 3 shows a schematic diagram of a sensor layout layer;
Figure 4 shows a schematic diagram of a two-layer sensor arrangement;
Figure 5 shows two schematic diagrams of sensor arrangements;
Figure 6 shows eight schematic diagrams of sensor arrangement; Figure 6-1 shows a timing diagram of the T1 cycle;
FIG 6-2 shows a timing diagram of a detection cycle T1-T7; FIG7 shows a schematic diagram of a top opening configured as a flat hole;
Figure 8 shows the top opening configuration as two schematic diagrams;
FIG 9 shows a schematic diagram of a device for measuring body diffusion substances with the addition of temperature and humidity sensors;
FIG 10 is a schematic diagram showing the projection of the top opening within the projection area of the bottom opening;
Figure 11 shows a schematic diagram of the connection between the bottom of the third opening and the bottom opening.

The technical solutions in the embodiments of the present invention will be described clearly and completely below with reference to the accompanying drawings in the embodiments of the present invention.

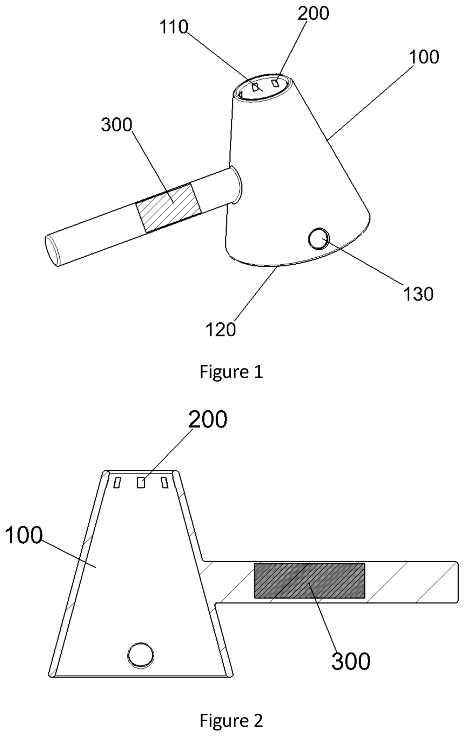

in FIG1 , a device for measuring a substance diffused in a body includes a measuring chamber 100 , a sensor 200 , and an evaluation device 300. The measuring chamber 100 has at least one top opening 110 for discharge and a bottom opening 120 for direct or indirect contact with the body, wherein the area of the top opening 110 is configured to be smaller than the bottom opening 120. The measuring chamber 100 also has at least one third opening 130. The third opening 130

It is used to make the measuring chamber 100 form an open bottom structure during measurement.

In FIG2, the sensor 200 is located on the peripheral wall of the measuring chamber 100 and is used to measure the concentration of the substance diffused by the body; the evaluation device 300 is coupled to the sensor 200 ; the peripheral wall may include the outer wall and / or the interior of the measuring chamber 100, wherein the preselected sensor 200 is arranged on the inner wall in increments.

Sensitivity.

In FIG 1, the present invention configures the top opening 110 of the measuring chamber 100 to be smaller than the bottom opening 110.

The opening 120 is provided, and a third opening 130 is provided at the bottom of the measuring chamber 100 to form a bottom open structure.

The airflow in the measuring chamber 100 is upward in a stable manner, avoiding up and down convection, thereby fundamentally resolving the contradiction and conflict with the theoretical basis of the diffusion law and improving the measurement accuracy.

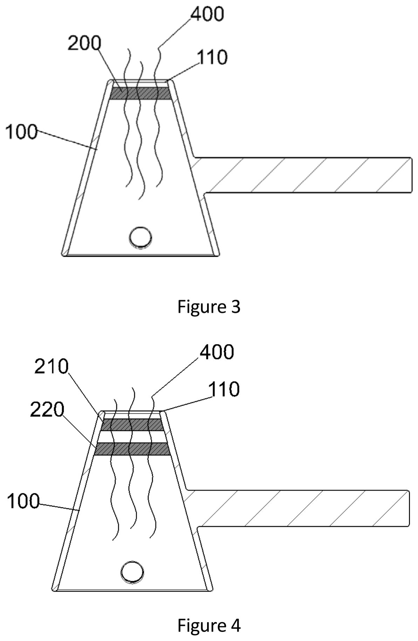

In FIG 3, taking the detection of water evaporation as an example, the evaporating water vapor flow 400 is concentrated at the top
The sensor 200 can be arranged around the top opening 110 of the measuring chamber 100 to facilitate

At the top opening 110. In addition, since a stable airflow is formed in the measuring chamber 100,
Sensors 200 can be configured to form a single layer of sensors arranged around the top opening 110.

Structure to achieve the purpose of simplifying the structure and saving costs; or, as shown in Figure 4, the number of sensors 200 is configured to be at least two, wherein each sensor 210, 220 is at a different distance from the body during measurement, thereby forming a multi-layer sensor structure with different heights around the top opening 110, thereby achieving the purpose of further improving the measurement accuracy .

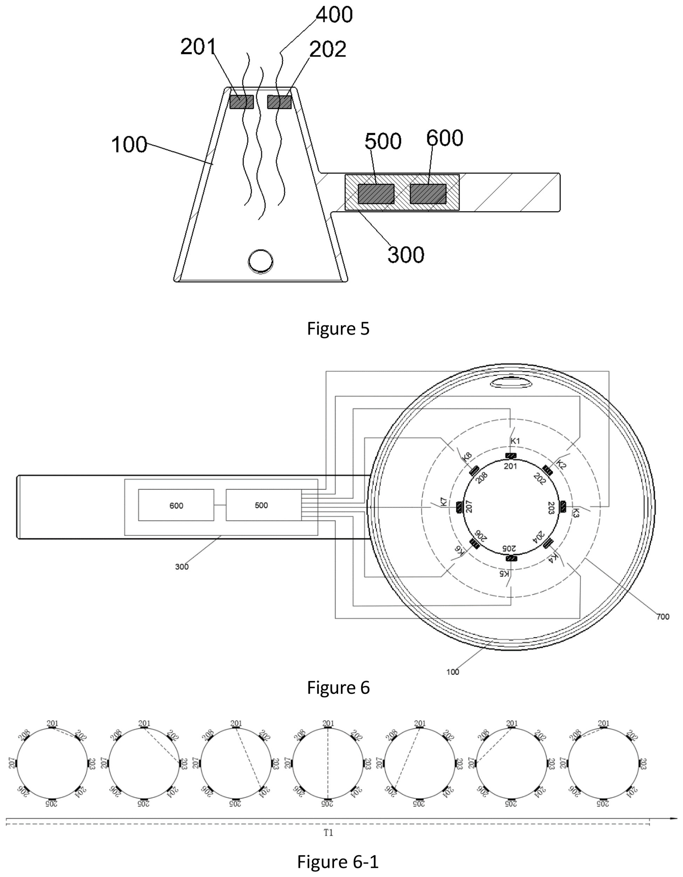

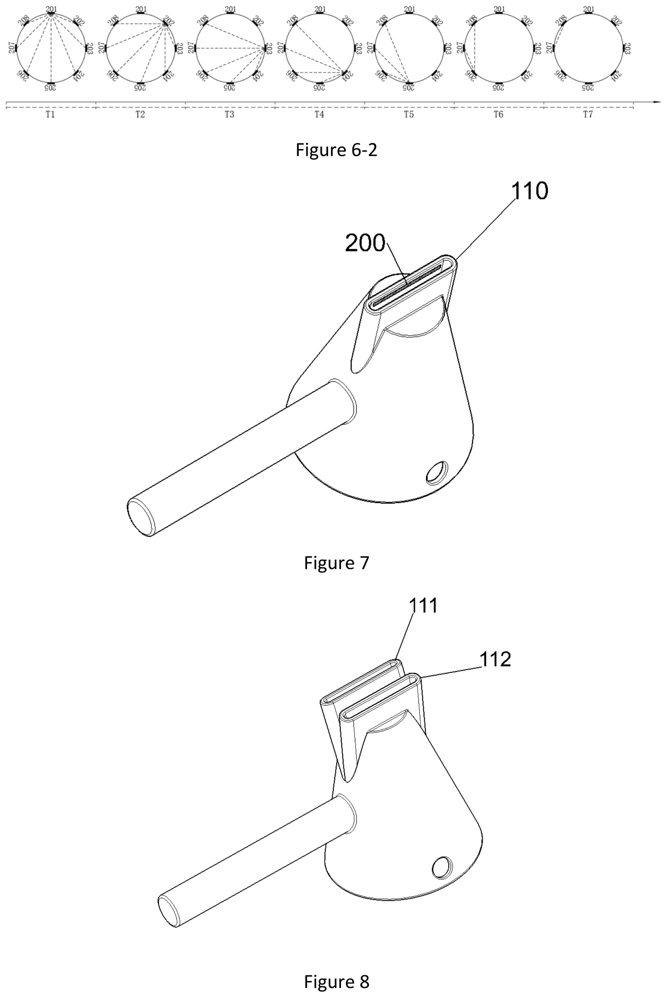

In FIG 5, the sensor is configured to include two electrodes 201 and 202. The evaluation device 300 is provided with a capacitance-to-digital conversion circuit (CDC) 500 and a processing module 600. The capacitance-to-digital conversion circuit 500 couples the electrodes 201 and 202 to obtain mutual capacitance. For the processing module 600, since the water vapor 400 passes through the mutual capacitance,
The capacitive electric field causes the dielectric in the electric field to change, resulting in a change in mutual capacitance. Therefore, the processing module 600 can
The change in mutual capacitance reflects the amount of water vapor 400 passing through the mutual capacitance electric field, and further outputs the concentration information of the substance diffused by the body. As shown in Figure 6, the sensor is configured to include eight electrodes 201, 202, 203, 204, 205, 206, 207, and 208. The capacitance-to-digital conversion circuit 500 couples each electrode through a switch array 700, and is used to use each electrode in the sensor as an excitation in turn to obtain the mutual capacitance between it and other electrodes in the layer. For example, as shown in Figure 6-2, a detection cycle includes T1-T7, a total of 7 detection cycles.

T1 period, electrode 201 is used as an excitation to obtain the mutual capacitance between electrode 201 and other electrodes in the layer. The specific detection method is shown in Figure 6-1 . The analog switches K1 and K2 are closed to detect the mutual capacitance between electrodes 201 and 202 , the analog switches K1 and K3 are closed to detect the mutual capacitance between electrodes 201 and 203 , the analog switches K1 and K4 are closed to detect the mutual capacitance between electrodes 201 and 204, and the analog switches K1 and K5 are closed to detect the mutual capacitance between electrodes 201 and 204.

The mutual capacitance between electrodes 201 and 205 is detected. The closed analog switches K1 and K6 detect the mutual capacitance between electrodes 201 and 206. The closed analog switches K1 and K7 detect the mutual capacitance between electrodes 201 and 207. The closed analog switches K1 and K8 detect the mutual capacitance between electrodes 201 and 208. As shown in Figure 6-2 , the electrode detection method during time T2-T7 is similar to that during time T1, with the number of mutual capacitances detected decreasing. This dynamic and synchronous detection of multiple pairs of mutual capacitances can further improve detection accuracy and sensitivity.
in FIG 7, the top opening 110 is configured as a flat hole, and the electrodes 200 are distributed on the flat edges of the flat hole on both sides. In this solution, the top opening area remains unchanged while the spacing between the electrodes can be further reduced, significantly optimizing both detection accuracy and sensitivity. Alternatively, as shown in FIG5 and FIG8, the top opening can be configured with two openings 111 and 112. The processing module 600 is configured to calculate the corresponding concentration information based on the collected data from the sensors at each top opening and perform a weighted average. By providing multiple openings, the total top opening area remains unchanged while the spacing between the electrodes at each opening is reduced. Accuracy can then be further improved through weighted averaging.

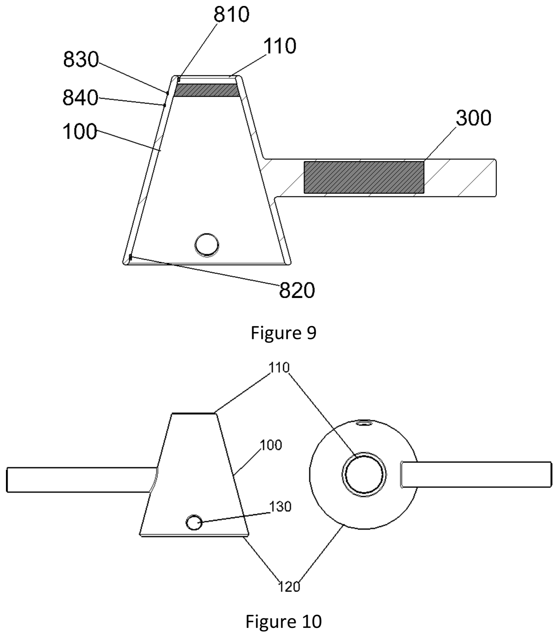

As shown in Figure 1, the temperature difference between the ambient temperature at the top opening and the body temperature (experimental environment human
The skin temperature of the human body is approximately 36 degrees Celsius, and the ambient temperature at the top opening is approximately 20 degrees Celsius , with a temperature difference of more than ten degrees between the two. This has an impact on the flow rate (temperature and humidity also affect mutual capacitance in the capacitive sensing solution), and the body temperature of different bodies (such as humans) varies slightly. Therefore, as shown in Figure 9 , a top temperature sensor 810 for collecting the ambient temperature at the top opening 110 and a bottom temperature sensor 820 for collecting the body temperature during the measurement period can be provided in the measurement chamber 100 ; the evaluation device 300 is coupled to the top temperature sensor 810 and the bottom temperature sensor 820 respectively to correct the concentration of the substance diffused from the body based on the temperature difference collected by the two , thereby eliminating the impact of the temperature difference during the measurement period. Furthermore, since the temperature and humidity in different environments are also different, the measurement benchmarks of the environments are different, introducing measurement errors. For this reason, the body diffusion substance measurement device is also provided with an ambient temperature sensor 830 and / or an ambient humidity sensor 840 located outside the measurement chamber. The evaluation device 300 is coupled to the ambient temperature sensor 830 and/ or the ambient humidity sensor 840 , and is used to correct the concentration of the substance diffused by the body based on the collected data of the ambient temperature sensor 830 and / or the ambient humidity sensor 840 , so as to eliminate the interference caused by changes in ambient temperature and humidity .

FIG 10, the top opening 110 is located on the top surface of the measurement chamber, and the bottom opening 120 is located on the bottom surface of the measurement chamber.

The projection of the top opening 110 is within the projection area of the bottom opening 120.
with the bottom opening 120 achieves a better flow stabilization and a less chaotic airflow. The measuring chamber 100 has a tapered structure that gradually narrows from the bottom to the top.

In FIG 10, the third opening 130 is used to open the bottom of the measurement chamber 100 during measurement.

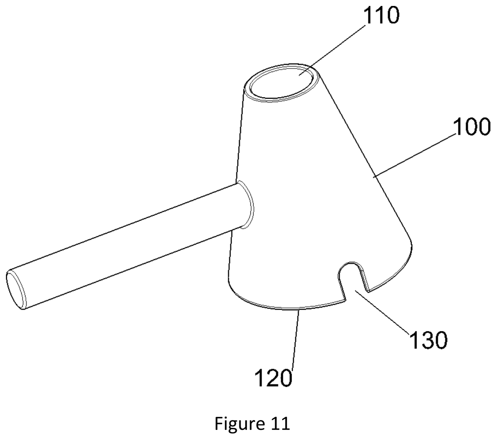

The structure can be in various ways. For example, as shown in FIG 11, by connecting the bottom of the third opening 130 to the bottom.

The opening 120 is connected, and the third opening 130 is set to be large enough so that the bottom opening is pressed against the body, such as a person.

When the skin of the body is on the body, the elastic skin of the body is not enough to completely block the third opening 130, thereby reaching the bottom.

In this solution, the elasticity of the skin of each body is different, and the third opening 130.

130 will also be different, so that the wind speed entering through the third opening 130 will be different and will also introduce an impact on the measurement. We hope that the area of the bottom opening is as similar as possible each time we measure it. Therefore, a gap can be set between the lower edge of the third opening 130 and the bottom opening 120 to prevent the skin of the body from being trapped in the third opening.
the opening 130 is to maintain the stability of the bottom opening area each time it is measured. In the present invention, the third opening 130.

The lower edge must be at least 1mm away from the bottom opening.

Finally, it should be noted that the above embodiments are only used to illustrate the technical solutions of the present invention, rather than to limit the scope of protection of the present invention . Although the present invention has been described in detail with reference to the preferred embodiments, those skilled in the art should understand that the technical solutions of the present invention may be modified or replaced by equivalents without departing from the essence and scope of the technical solutions of the present invention.

## Claims

1. A device for measuring diffusible substances in a body, comprising:
a measuring chamber (100) having at least one top opening (110) for discharge and a bottom opening (120) for direct or indirect contact with the body;
a sensor (200), located on a peripheral wall of the measuring chamber (100), for measuring the concentration of a substance diffused from the body;
an evaluation device (300), coupled to the sensor (200);
**characterized in that**:
an area of the top opening (110) is configured to be smaller than an area of the bottom opening (120);
the measuring chamber (100) is further provided with at least one third opening (130), wherein the third opening (130) is for forming the measuring chamber (100) into an open-bottom structure during measurement.

2. The device for measuring diffusible substances in a body according to claim 1, wherein the sensor (200) is arranged around the top opening (110) of the measuring chamber (100).

3. The device for measuring diffusible substances in a body according to claim 2, wherein:
the number of the sensors (200) is configured as one to form a single-layer sensor structure; or
the number of the sensors (200) is configured to be at least two, wherein the respective sensors (200) are at different distances from the body during measurement to form a multi-layer sensor structure.

4. The device for measuring diffusible substances in a body according to claim 3, wherein:
the sensor (200) is configured to include at least two electrodes (201);
the evaluation device (300) is provided with a capacitance-to-digital conversion circuit (500) and a processing module (600), wherein the capacitance-to-digital conversion circuit (500) is coupled to each electrode (201) to obtain a mutual capacitance;
the processing module (600) is configured to output concentration information of a substance diffused from the body according to the mutual capacitance.

5. The device for measuring diffusible substances in a body according to claim 4, wherein:
the sensor (200) is configured to include at least three electrodes (201);
the capacitance-to-digital conversion circuit (500) couples the electrodes (201) via a switch array (700), and is configured to sequentially use each electrode (201) in the sensor (200) as an excitation to obtain the mutual capacitance between the electrode (201) and other electrodes (201) in the layer.

6. The device for measuring diffusible substances in a body according to claim 4, 5 or 6, wherein:
a ratio of an area of the top opening (110) to an area of the bottom opening (120) is configured to be less than 0.6.

7. The device for measuring diffusible substances in a body according to claim 4, 5 or 6, wherein the top opening (110) is configured as a flat hole, and the electrodes (201) are distributed on flat edges on both sides of the flat hole.

8. The device for measuring diffusible substances in a body according to claim 4, 5 or 6, wherein:
the top opening (110) is configured to include at least two;
the processing module (600) is configured to calculate corresponding concentration information based on collected data of each sensor (200) of each top opening (110) and perform weighted averaging.

9. The device for measuring diffusible substances in a body according to claim 1 or 4, **characterized in that**:
the measuring chamber (100) is provided with a top temperature sensor (810) for collecting an ambient temperature at the top opening (110), and a bottom temperature sensor (820) for collecting a body temperature during a measurement period;
the evaluation device (300) is coupled to the top temperature sensor (810) and the bottom temperature sensor (820) respectively, and is configured to correct the concentration of the substance diffused from the body according to a temperature difference collected by the two.

10. The device for measuring diffusible substances in a body according to claim 9, **characterized in that**:
the device for measuring diffusible substances in a body is further provided with an ambient temperature sensor (830) and/or an ambient humidity sensor (840) located outside the measuring chamber (100);
the evaluation device (300) is coupled to the ambient temperature sensor (830) and/or the ambient humidity sensor (840), and is configured to correct the concentration of the substance diffused from the body according to collected data of the ambient temperature sensor (830) and/or the ambient humidity sensor (840).

11. The device for measuring diffusible substances in a body according to claim 1, wherein the top opening (110) is located on a top surface of the measuring chamber (100), the bottom opening (120) is located on a bottom surface of the measuring chamber (100), and a projection of the top opening (110) is within a projection area of the bottom opening (120).

12. The device for measuring diffusible substances in a body according to claim 11, wherein the measuring chamber (100) is in a tapered structure that gradually narrows from a bottom to a top.

13. The device for measuring diffusible substances in a body according to claim 11, wherein a distance exists between a lower edge of the third opening (130) and the bottom opening (120).

14. The device for measuring diffusible substances in a body according to claim 1, wherein:
the diffusible substance is configured as diffused water vapor.
